# EUROPEAN PATENT SPECIFICATION

(11) **EP 2 576 010 B1**
(45) Date of publication and mention of the grant of the patent: **31.08.2016**
(21) Application number: 11763833.8
(22) Date of filing: 13.05.2011
(51) Int. Cl.: C10L 3/10, B01D 53/22, B01D 61/38

(54) **A PROCESS FOR ENRICHING OF METHANE IN BIOGAS FROM SEWERAGE PLANTS OR AGRICULTURAL BASIC INDUSTRIES IN METHANE AND AN APPARATUS FOR CARRYING OUT THE SAME**
VERFAHREN ZUR ANREICHERUNG VON METHAN MIT EINEM BIOGAS AUS KANALISATIONSANLAGEN ODER LANDWIRTSCHAFTLICHEN BASISINDUSTRIEN SOWIE VORRICHTUNG ZUR AUSFÜHRUNG DIESES VERFAHRENS
PROCÉDÉ D'ENRICHISSEMENT EN MÉTHANE DE BIOGAZ ISSU DE STATIONS D'ÉPURATION OU D'INDUSTRIES AGRICOLES ET APPAREIL POUR LA MISE EN OEUVRE DU PROCÉDÉ

(30) Priority: 02.06.2010 CZ 20100437
(43) Date of publication of application: 10.04.2013
(73) Proprietor: Ceská hlava s.r.o., 267 12 Svatý Jan pod Skalou (CZ)
(72) Inventor: IZÁK, Pavel, 190 16 Praha 9 (CZ); POLONCARZOVÁ, Magda, 412 01 Litomerice (CZ); VEJRAZKA, Jirí, 165 02 Praha 6 (CZ)
(74) Representative: Plicka, Josef
(86) International application number: PCT/CZ2011/000052
(87) International publication number: WO 2011/150899

(56) References cited:
- EP-A1- 1 775 015
- US-A- 4 737 166
- US-A- 4 961 758
- US-B1- 6 958 085
- ASIM K. GUHA ET AL: "Gas separation modes in a hollow fiber contained liquid membrane permeator", INDUSTRIAL & ENGINEERING CHEMISTRY RESEARCH, vol. 31, no. 2, 1 February 1992 (1992-02-01), pages 593-604, XP55019538, ISSN: 0888-5885, DOI: 10.1021/ie00002a022
- KIMURA S G ET AL: "FUEL GAS PURIFICATION WITH PERMSELECTIVE MEMBRANES", SEPARATION SCIENCE AND TECHNOLOGY, DEKKER, NEW YORK, NY, US, vol. 15, no. 4, 1 January 1980 (1980-01-01), pages 1115-1133, XP001060365, ISSN: 0149-6395, DOI: 10.1080/01496398008076290

## Description

### Field of Invention

The present invention relates to a process for enriching the biogas from sewerage plants or agricultural basic industries in methane. The process comprises introducing the biogas to one side of a liquid water membrane which is immobilized in/on a porous support. A stream enriched in methane and depleted in carbon dioxide and further undesirable components contained in the biogas is obtained as a retentate and a stream depleted in methane and enriched in carbon dioxide and further components is obtained as a permeate. The invention also relates to an apparatus for carrying out said process. The apparatus comprises a membrane separator, which is divided by a water liquid membrane immobilized in/on a porous support, into a retentate space and a permeate space. The retentate space is connected with supply fittings for the biogas and discharge fittings for the retentate stream whereas the permeate space is connected with discharge fittings for the permeate stream. The proposed separation process makes possible to use biogas in a more effective manner.

### Background Prior Art

Individual gas components have been separated by different separation techniques. These techniques include e.g. membrane processes, adsoprption, absorption and cryogenic distillation. Enriching the biogas from sewerage plants landfill or agricultural basic industries in methane has acquired great economic importance.

Biogas is a gaseous mixture which is formed by anaerobic digestion of the residues of plant and animal origin. During said process, biomass is decomposed by the action of bacteria in the absence of oxygen to form particularly methane, carbon dioxide and small amounts of further gases, such as ammonia or sulfane. The methane content of biogas ranges from 50 to 70 % by volume (W. Kossmann, U. Pönitz et. al.: Biogas Basics, Biogas Digest vol.1, project of Information and Advisory Service on Appropriate Technology (ISAT)).

It is just methane, which is also the main component of natural gas, that makes possible to use biogas as an ecological fuel.

At present, biogas is only used in the so-called cogeneration units (Combined heat and power plant) for the combined production of electricity and heat, in the Czech republic. However, in other countries, such as Sweden or Germany, methods of processing biogas so as to obtain a product similar to natural gas, which could be used for instance as a fuel for transportation vehicles, have been put into operation. (M. Persson, O. Jonssen, A. Wellinger, Biogas Upgrading to Vehicle Fuel Standards and Grid Injection. IEA Bioenergy task 37, Impressium, 2006)

The above mentioned method of biogas processing requires however a multistep procedure which comprises drying biogas and removing aggressive gases therefrom, as well as carbon dioxide separation. The following procedures are e.g. used during said purification (compare the citation M. Persson et al., above and moreover, also M. Persson: Evaluation of Upgrading Techniques for Biogas. School of Environmental Enginering, Lund University, Sweden, 2003):
- drying - cryogenic units, adsorption on silicagel or lyophilization;
- sulfane removal - scrubbing with NaOH solution, adsorption on active carbon with further oxidation,
- ammonia removal - absorption in sulphuric acid;
- carbon dioxide separation - water absorption, lyophilization, PSA, membrane separation with the use of polymeric membranes.

It is just the membrane separation that seems to be a promising method of upgrading biogas. The term membrane separation is used to designate a process in which a mixture is separated on the basis of different permeability of individual components across a selective membrane by means of which two spaces are separated from one another. The transport of the components across the membrane is influenced by their physical and chemical properties, and the interactions of the components between each other and the membrane, itself. With the use of porous membranes the components are most frequently separated on the basis of their particle size. With the use of non-porous membranes the transport properties are particularly dependent on the solubility of the components within the membrane or optional possibility of chemical interactions between the individual components and the membrane, and the diffusion coefficients of the components in the membrane.

In now-a-days practice the separation of gaseous mixtures is mostly carried out with the help of non-porous membranes made of polymeric materials (R. Barker: Recent Developements in Membrane Vapour Separation System. Membrane Technology 114 (1999) 9-12). Their main disadvantage is that they become contaminated by toxic materials, which are often present in the biogas from sewerage plants or agricultural biogas (see Table 1).

**Table 1**

| Concentration of some hydrocarbon derivatives and organo-sulphur compounds in biogas (F. Straka,: Bioplyn. Praha, GAS (2006) 706. | |
|---|---|
| **Component** | **c[ppm]** |
| methanol | 2-210 |
| ethanol | 16-1450 |
| 1- propanol | 4.1-630 |
| 1- butanol | 2.3- 73 |
| acetone | 0.27- 4.1 |
| butyl acetate | 60 |
| acetic acid | 0.06- 3.4 |
| butanoic acid | 0.02- 6.8 |
| methanethiol | 0.1-30 |
| dimethyl sulphide | 1.6-4 |
| dimethyl disulphide | 0.02- 40 |
| carbon disulphide | 0.5- 22 |
| ethanethiol | 0- 20 |

Due to such contamination the separation membranes lose their original selectivity and the permeation flow is also reduced. Contaminated membranes have to be replaced and moreover, they must be handled as toxic waste, which is very expensive.

Another possible approach is gas separation with the help of so called liquid membranes (J.E. Bara, S. Lessmann, C. J. Gabriel, E. S. Hatakeyama, R. D. Noble, D. L. Gin, Synthesis and Performance of Polymerizable Room-Temperature Ionic Liquids as Gas Separation Membranes, Ind. Eng. Chem. Res. 46 (2007) 5397). At present, the liquid membrane technology is being tested for the separation of carbon dioxide from methane. The most recent trend in this respect are so-called anchored ionic membranes (J. E. Bara, S. E. Hatakeyama, D. L. Gin, R. D. Noble; Improving CO2 Permeability in Polymerized Room-temperature Ionic Liquid Gas Separation Membranes through the Formation of a Solid Composite with a Room-temperature Ionic Liquid, Polym. Adv. Technol.; 19 (2008) 1415.

Immobilized ionic membranes comprise ionic liquids which, owing to their high selectivity and molecular diffusion, as well as negligible vapour tension, represent ideal materials for the preparation of liquid membranes. (J. E. Bara , Ch. J. Gabriel , E. S. Hatakeyamaa, T. K. Carlisle: Improving CO2 Selectivity in Polymerized Room-temperature Ionic Liquid Gas Separation Membranes through Incorporation of Polar Substituents, Journal of Membrane Science 321 (2008) 3-7). In general, the main diadvantage of the liquid membranes consists in that their stability depends on capillary forces which are not always capable of resisting to the long-termed (or even operational) duty. Moreover, ionic liquids more or less absorb moisture, which leads to relatively quick breakdown of the membrane structure and stability.

The hitherto conducted investigations have shown that it is the solubility of a separated component in a liquid membrane that is critical for the selectivity of a liquid membrane (P. Izák, L.Bartovská, K. Friess, M. 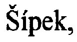 P. Uchytil; Comparison of Various Models Flat for Transport of Binary Mixtures through Dense Polymer Membrane, *Polymer,* 44 (2003) 2679-2687).

A suitable liquid for the separation has to comply with the requirement that the separated gases be at least by one order of magnitude more soluble in the selected liquid than methane which is the predominant component of biogas. Hence, the selection of a liquid for the impregnation of a porous membrane is a key factor.

For proper membrane function, an appropriate selection of the so-called support, in which the selected liquid is anchored, is very important, in particular in view of the rigidity and the stability of the system. Moreover, high affinity between the liquid and the porous support is a must.

The article of Kimura & al. (Sep.Sci.Tech., Vol.15(4), p.1115) discloses a process for enriching a biogas in methane, which comprises introducing the biogas to one side of a liquid membrane which is immobilized on a porous hydrophilic support, the liquid membrane consisting of an aqueous carbonate solution.

The hitherto known liquid membranes for the separation of biogas aimed at obtaining a stream enriched in methane and depleted in carbon dioxide and detrimental components, such as sulphur-containing components, have suffered from certain complexity and expensiveness of the used liquid systems, e.g. consisting of compounds which at the same time may contain hydroxy and amino groups, such as aminic derivatives of glycols, etc. If water systems are used, the liquid membrane need not be sufficiently stable. Stability may be impaired by a varying amount of the liquid embedded in the membrane support. In water systems such undesirable variations can be caused by the fact that under the operational conditions water from the membrane is evaporated the separated gas whereby the membrane is in fact getting dried. The membrane stability is however very important from the point of view of the continuous operation of a separation unit.

### Subject Matter of Invention

The above mentioned disadvantages are solved by a process and an apparatus according to the invention, which are described below.

The subject matter of the present invention is a process for enriching the biogas from sewerage plants, landfill or agricultural basic industries in methane, which comprises introducing the biogas to one side of a liquid water membrane which is immobilized in/on a porous hydrophilic support. A stream enriched in methane and depleted in carbon dioxide, hydrogen sulfide and further undesirable components contained in the biogas is obtained as a retentate, and a stream depleted in methane and enriched in carbon dioxide and further undesirable components contained in the biogas components is obtained as a permeate. The improvement of said process lies in that as liquid membrane water alone is used, and the losses of water from said membrane, which are caused by driving away said water in said permeate stream, are compensated by saturating said introduced biogas with steam at a temperature which is higher than the temperature at which said membrane separation takes place whereupon said steam is condensed when said biogas is cooled in said membrane (below dew point of the introduced biogas).

In general, as the porous hydrophilic supports any materials having high affinity to water in the pores of which vapor with which the introduced biogas has been saturated can spontaneously condense as soon as it is cooled to the temperature below its dew point. Such materials can include polymer porous membranes e.g. made of hydrophilized polytetrafluoroethylene, polyamide or other hydrophilic polymers or ceramic membranes e.g. on the basis of alumina.

In a preferred embodiment of said process the permeation takes place into sweeping gas. In another embodiment reduced pressure can be maintained on the permeate side of a permeation cell.

The subject matter of the present invention also includes an apparatus for carrying out said process. Said apparatus comprises a membrane separator, which is divided by a liquid water membrane immobilized in/on a porous hydrophilic support, into a retentate space and a permeate space. Said retentate space is connected with supply fittings for said gaseous mixture and discharge fittings for said retentate stream whereas said permeate space is connected with discharge fittings for said permeate stream. A saturator for saturating said biogas with vapor is inserted into the supply tubing for the introduced biogas.

For the correct function of both the process and the apparatus according to the invention it is critical that the solubility of the separated gases in the separation liquid may be substantially different. This prerequisite is duly met in the case of water as a separation membrane for the system methane/carbon dioxide. Moreover, the fact that no further components need to be added to said water substantially adds to cost reduction. For the correct function of the system it should be also ensured that water content is maintained in the the pores of the support. To this end, in a preliminary step the biogas introduced to the membrane is saturated with the separation liquid., i.e. water. The saturation takes place at a temperature, which is higher than the temperature in the membrane separator in which the liquid water membrane immobilized in/on the pores of the support is located. The temperature difference between the introduced biogas and the water membrane is such that spontaneous condensation of water in/on the pores may take place. The amont of water in the membrane can be controlled by adjusting the temperature, at which biogass is saturated by water.

The advantages of the proposed separation method can be summarized as follows:
1. A very low cost of the separation medium.
2. The continuous regeneration of the membrane liquid in the course of the separation process.
3. The prevention of accumulation of residual toxic substances contained in the biogas. As a consequence the pretreatment with a sorbent and further handling problems with the used sorbent, which shall be disposed off as a toxic waste, both of which are demanding from the economic point of view, may be avoided.

### Brief Description of Drawings

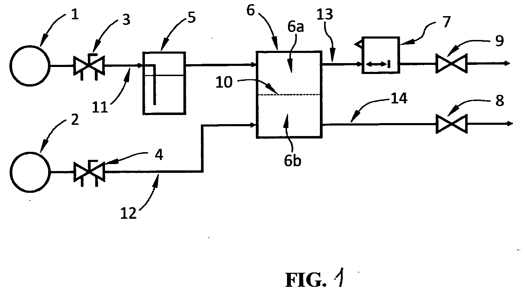

The attached figure contains a schematic diagram of an example of an apparatus according to the invention.

The process according to the invention is illustrated by the following working examples which are carried out in the apparatus depicted in the attached figure. The examples are only illustrative, and they do not limit the claimed scope in any respect.

### Working Examples

### Example 1

### Enrichment of model biogas

The real biogas from sewerage plants (represented by a ternary mixture (70% vol. methane, 30% vol. carbon dioxide a 2800 ppm sulfane) is introduced from a pressure container 1 of biogas over a flow controller 3 of the biogas into a saturator 5, in which the biogas is saturated with steam, and then into a membrane separator 6. The temperature in the saturator 5 of the biogas with steam is higher than the temperature in the membrane separator 6, in which a water impregnated porous support of hydrophilized polytetrafluoroethylene is mounted. Partial gas flows through this condensing water membrane at the pressure over the membrane 350 kPa are as follows: J_{CO2} = 22.3 1/(m²h), J_{CH4} = 1.26 1/(m²h) a J_{H2S} = 0.08 1/(m²h).

**Table 2**

| Separation of model biogas with the help of a condensing water membrane | | | | |
|---|---|---|---|---|
| Pressure over the membrane 350kPa | Feed | Permeate | Permeation flow (1/m²/h) | Gas flow over the membrane (ml/min) |
| Methane | 70 vol. % | 5.4 vol. % | 22.3 | 24 |
| Carbon dioxide | 29.7 vol. % | 94.2 vol. % | 1.26 | 10 |
| Sulfane | 2800 ppm | 3429 ppm | 0.08 | 10 |

The temperature difference is such that spontaneous water condensation in the pores of the porous membrane may take place. The driving force of the separation process is the different concentration of individual gas components and the overall pressure over and under the membrane. The permeation takes place into the carrier gas, which is nitrogen, introduced below the membrane from a pressure container 2 of the carrier gas over the carrier gas flow controller 4. Optimal total pressure over the membrane is controlled by a reverse pressure controller 7.

### Example 2

### Enrichment of real biogas

For the separation a real biogas from a sewerage plant is used. The composition of the biogas is shown in Table 3. The biogas is introduced from a pressure container 1 of biogas over a flow controller 3 of the biogas at a flow rate 10 ml/min into a saturator 5, in which the biogas is saturated with vapour which is formed from the introduced tap water, and then into a membrane separator 6. The temperature in the saturator 5 of the biogas with steam is higher (27 °C) than the temperature in the membrane separator 6 (14 °C). In the membrane separator 6 a membrane 10 of hydrophilic teflon (having pores 0.1 µm, thickness 30 µm and porosity 80 %), impregnated with tap water, is mounted. The effective area of the membrane having the radius of 13 cm is 132,7 cm². The permeation is carried out into the carrier gas, which is nitrogen. The conducted experiments show (see Table 3) that methane can be efficiently concentrated with the use of a porous membrane of hydrophilic teflon in which water condensation takes place.

**Table 3**

| Enrichment of real biogas in methane with the use of a condensing water membrane | | | | |
|---|---|---|---|---|
| Sample designation | amount | feed | retentate | permeate |
| Aromatic hydrocarbons | | | | |
| toluene | mg/m³ | 38.6 | 6.26 | 15.2 |
| ethylbenzene | mg/m³ | 10.9 | <0,07 | 2 |
| xylenes | mg/m³ | 37.6 | 1.66 | 6.6 |
| Total aromatic hydrocarbons | mg/m³ | 87.1 | 7.92 | 23.8 |

| Chlorinated and aliphatic hydrocarbons | | | | |
|---|---|---|---|---|
| cis-dichloroethene | mg/m³ | 1.57 | <0,10 | <0,10 |
| trichloroethene | mg/m³ | 2.54 | 0.960 | 1.74 |
| tetrachloroethene | mg/m³ | 4.15 | 1.04 | 1.29 |
| Total chlorinated and aliphatic hydrocarbons | mg/m³ | 8.26 | 2 | 3.8 |

| BASIC GAS ANALYSIS | | | | |
|---|---|---|---|---|
| carbon dioxide | % v/v | 30 | 21.3 | 86 |
| methane | % v/v | 69 | 76 | 14 |
| sulfane | mg/m³ | 7,95 | <0,91 | <0,61 |

| SILOXANes | | | | |
|---|---|---|---|---|
| L3 - oktamethyltrisiloxane | mg/m³ | 4.13 | <0,1 | <0,1 |
| D4 - oktamethylcyclotetrasiloxane | mg/m³ | 14.4 | <0,1 | <0,1 |
| L4 - decamethyltetrasiloxane | mg/m³ | 2.2 | <0,1 | <0,1 |
| D5 - decamethylcyclopentasiloxane | mg/m³ | 109.6 | 9.1 | 13 |
| Total siloxanes | mg/m³ | 130.3 | 9.1 | 13 |

The temperature difference (13 °C) is such that spontaneous water condensation in the pores of the porous membrane may take place. The driving force of the separation process is the different concentration of individual gas components and the overall pressure over and under the membrane. The permeation takes place into the carrier gas, which is nitrogen, introduced below the membrane from a pressure container 2 of the carrier gas over the carrier gas flow controller 4. Optimal total pressure over the membrane is controlled by a reverse pressure controller 7.

### List of Reference Numerals

1- pressure container for gaseous mixture
2 - pressure container for carrier gas
3 - gaseous mixture flow controller
4 - carrier gas flow controller
5 - saturator of gaseous mixture with vapour of selected liquid
6 - membrane separator
6a - retentate space
6b - permeate space
7 - reverse pressure controller
8 - permeate discharge valve
9 - valve for retentate effluent
10 - hydrophilic porous support
11 - 14 - piping
11 - feeding pipe for gaseous mixture
12 - feeding pipe for carrier gas
13 - discharging pipe for retentate
14 - discharging pipe for permeate

## Claims

1. A process for enriching the biogas from sewerage plants or agricultural basic industries in methane, which comprises introducing the biogas to one side of an liquid water membrane which is immobilized in/on a porous hydrophilic support, wherein a stream enriched in methane and depleted in carbon dioxide, sulfane and further undesirable components contained in the biogas is obtained as a retentate, and a stream depleted in methane and enriched in carbon dioxide and further undesirable components contained in the biogas components is obtained as a permeate and the losses of water from said membrane, which are caused by driving away said water in said permeate stream, are compensated by saturating said introduced biogas with steam at a temperature which is higher than the temperature at which said membrane separation takes place whereupon said steam is condensed when said biogas is cooled in said membrane, **characterized in that** water alone is used as liquid membrane

2. A process according to claim 1, wherein said permeation is carried out into a sweeping gas.

3. An apparatus for carrying out the process according to claim 1 or 2 which comprises a membrane separator (6), which is divided by a liquid water membrane immobilized in a porous hydrophilic support, into a retentate space (6a) and a permeate space (6b), said retentate space (6a) being connected with supply fittings (1, 3, 5, 11) for said biogas and discharge fittings (13, 7, 9) for said retentate stream whereas said permeate space is connected with supply fittings (2, 4, 12) for carrier gas and discharge fittings (8, 14) for said permeate stream, wherein a saturator (5) for saturating said biogas with said steam is inserted into the supply tubing (11) for the introduced biogas, **characterized in that** the liquid membrane consists of water alone.

## Patentansprüche

1. Verfahren zur Anreichung von Biogas aus Abwasserreinigungsanlage oder aus primären Landwirtschaftsindustrien mit Methan, welches die Einführung von Biogas an einer Seite der flüssigen Wassermembrane, die in/auf einem hydrophilen porösen Support unbeweglich befestigt ist, umfasst, in welchem ein methanangereichter Strom und von Kohlendioxid, Sulfan und weiteren unerwünschen, in Biogas enthaltenen Komponenten befreiter Strom als Retentat vorliegt, sowie ein Strom befreit von Methan und durch Kohlendioxid und weiteren unerwünschen in Biogas enthaltenen Komponenten als Penneat angereichert ist, wobei die Wasserverluste aus der erwähnten Membrane, welche durch die Wasserableitung in den angegebenen Permeatstrom verursacht waren, durch die Saturation des eingeführten Biogases mit Dampf, dessen Temperatur höher ist als die Temperatur der durchgefühten Separation der Membrane, kompensiert sind, wonach der erwähnte Dampf kondensiert wird, wenn das erwähnte Biogas in der genannten Membrane abgekühlt wurde, **dadurch gekennzeichnet, dass** nur Wasser in der Flüssigkeitsmembrane verwendet wird.

2. Verfahren gemäs dem Patentanspruch 1, in welchem die erwähnte Permeation in dem Trägergas durchläuft.

3. Gerät zur Durchführung des Verfahren gemäss dem Patentanspruch 1 oder 2 mit einem Membranseparator (6), welcher durch eine Wassermembrane, die in einem hydrophilen porösen Support unbeweglich befestigt ist, in einen Retentatraum (6a) und einen Permeatraum (6b) getrennt ist, wobei der erwähnte Retentatraum (6a) durch Eintrittsfitkinge (1, 3, 5, 11.) für das Biogas und durch Austrittsfittinge (13, 7, 6) für den erwähnten Retentatstrom verbunden ist, wobei der erwähnte Permeatraum durch Eintrittsfittinge (2, 4, 12) für das Trägergas und durch Austrittsfittinge (8, 14) für den Permeatstrom verbunden ist, wobei ein Saturator (5) für die Saturation des erwähnten Biogases mit dem angegebenen Dampf in die Zuführungsleitung (11) für das eingeführte Biogas verbunden ist, **dadurch gekennzeichnet, dass** die Flüssigkeitsmembrane nur aus Wasser besteht.

## Revendications

1. Procédé pour enrichir un gaz de fumier issu d'une station d'épuration des eaux résiduaires ou d'industrie agricole primaire de méthane, lequel procédé introduit le gaz de fumier dans une face d'une membrane d'eau liquide laquelle est immobilisée dans/sur un support hydrophile poreux, dans lequel un courant enrichi de méthane et appauvri d'oxide carbonique, de sulfane et d'autres composants indésirables contenus dans le gaz de fumier est obtenu comme un rétentat et un courant appauvri de méthane et enrichi d'oxide carbonique et d'autres composants indésirables contenus dans le gaz de fumier est obtenu comme un perméat et des pertes d'eau de la membrane mentionnée lesquelles sont causées par un découlement de l'eau dans le courant perméat sont compensées par une saturation du gaz de fumier introduit avec une vapeur de température plus élévée que la température en laquelle une séparation par ladite membrane est réalisée, ce qui est suivi par la condensation de la vapeur lorsque ledit gaz de fumier est refroidi dans la membrane, **caractérisé en ce que** l'eau seulement est utilisée comme ladite membrane liquide.

2. Procédé selon la revendication 1, dans lequel on réalise ladite perméation dans le gaz porteur.

3. Appareil pour la mise en oeuvre du procédé selon la revendication 1 ou 2 lequel contient un séparateur de membrane (6) qui est divisé par la membrane d'eau liquide immobilisée dans le support hydrophile poreux dans un espace rétentat (6a) et dans un espace perméat (6b) lequel espace rétentat (6a) est est lié avec des raccords d'alimentation (1, 3, 5, 11) pour ledit gaz de fumier et avec des raccords d'évacuation (13, 7, 9) pour ledit courant rétentat, tandis que ledit espace perméat est lié avec des raccords d'alimentation (2, 4, 12) pour le gaz porteur et avec des raccords d'évacuation (8,14) pour ledit courant perméat, dans lequel un saturateur (5) destiné pour réaliser la saturation du gaz de fumier avec ladite vapeur est introduit dans la conduite d'alimentation (11) pour le gaz de fumier, **caractérisé en ce que** la membrane liquide consiste seulement de l'eau.
